# EUROPÄISCHE PATENTSCHRIFT

(11) **EP 1 947 166 B1**
(45) Veröffentlichungstag und Bekanntmachung des Hinweises auf die Patenterteilung: **19.01.2011**
(21) Anmeldenummer: 07121171.8
(22) Anmeldetag: 21.11.2007
(51) Int. Cl.: C11B 9/00, A61K 8/35, C11D 3/50

(54) **Mischungen von 3-(4-Methyl-cyclohex-3-enyl)-butyraldehyd und Ambro-cenide sowie deren Verwendungen und Verfahren**
Mixtures of 3-(4-methyl-cyclohex-3-enyl)-butyraldehyde and Ambrocenide and their uses and method
Mélanges de 3-(4-méthyl-cyclohex-3-enyl)-butyraldehyde et d'Ambrocenide, et ses utilisations et procédé

(30) Priorität: 02.01.2007 EP 07100015
(43) Veröffentlichungstag der Anmeldung: 23.07.2008
(73) Patentinhaber: Symrise AG, 37603 Holzminden (DE)
(72) Erfinder: Eh, Marcus, 37603, Holzminden (DE); Naraschkewitz, Fred, 21244, Buchholz Nordheide (DE)
(74) Vertreter: Eisenführ, Speiser & Partner

(56) Entgegenhaltungen:
- EP-A1- 0 857 723
- US-A- 2 710 825

## Beschreibung

Die vorliegende Erfindung betrifft gemäß einem ersten Aspekt Mischungen umfassend oder bestehend aus 3-(4-Methyl-cyclohex-3-enyl)-butyraldehyd (Limonenal) sowie (4aR,5R,7aS,9R)-Octahydro-2,2,5,8,8,9a-hexamethyl-4H-4a,9-Methanoazuleno(5,6-d)-1,3-dioxol) (CAS Nr. 211299-54-6; nachfolgend: Ambrocenide). Ein weiterer Aspekt betrifft die Verwendung von Ambrocenide® zur Maskierung einer unerwünschten Geruchsnote des Limonenal sowie entsprechende Verfahren zur Maskierung der besagten Geruchsnote des Limonenal. Schließlich betrifft die Erfindung auch die Verwendung einer erfindungsgemäßen Mischung als Riechstoff mit frischer, citrusartiger Note.

Ambrocenide® besitzt die folgende Struktur, siehe auch EP 0 857 723:

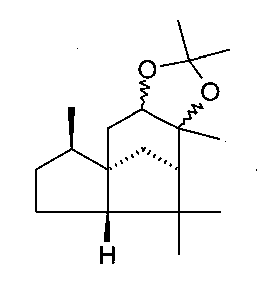

Die geschlängelten Linien können dabei unabhängig voneinander alpha- oder beta-Konfiguration bedeuten. Ambrocenide® kann ein, zwei, drei oder sämtliche der folgenden Diastereoisomere umfassen:

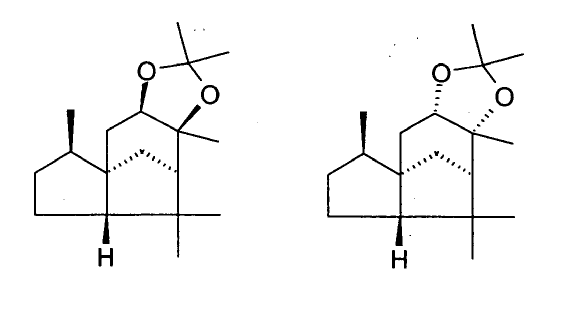

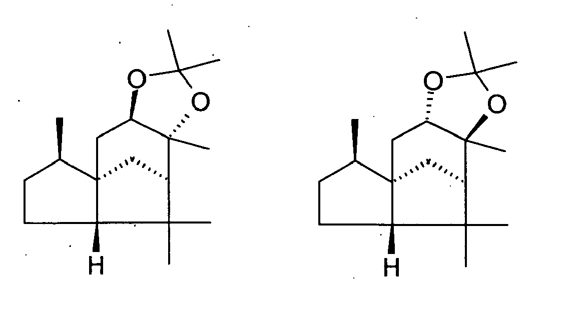

3-(4-Methyl-cyclohex-3-enyl)-butyraldehyd (Limonenal) ist bereits aus dem Stand der Technik bekannt:

In US 2,584,539 wird ausgehend von Limonen die Hydroformylierungsreaktion zu 3-(4-Methyl-cyclohex-3-enyl)-butyraldehyd beschrieben. Der Geruch von 3-(4-Methyl-cyclohex-3-enyl)-butyraldehyd wird als angenehm und langanhaltend beschrieben, Limonenal soll in Mischung mit oder als Ersatz für Hydroxycitronellal in Parfümmischungen verwendet werden. Insoweit ist anzumerken, dass Hydroxycitronellal einen blumigen Geruch besitzt.

US 2,710,825 beschreibt die Herstellung von unter anderem 3-(4-Methylcyclohex-3-enyl)-butyraldehyd aus Limonen. Der Geruch von 3-(4-Methylcyclohex-3-enyl)-butyraldehyd wird als stärker und länger anhaftend als der von Citral beschrieben und ausgeführt, dass der Butyraldehyd (Limonenal) zusätzlich eine grüne oder frische Note verleiht.

EP 011 272 A3 beschreibt die Herstellung von Aldehyden durch Hydroformylierung von Olefinen. Unter anderem wird die Herstellung von 3-(4-Methyl-cyclohex-3-enyl)-butyraldehyd durch Hydroformylierung von Limonen beschrieben. Der Geruch von 3-(4-Methyl-cyclohex-3-enyl)-butyraldehyd wird als Agrumen- und Rhabarber-Note angegeben. Erwähnt wird weiter, dass diese Verbindung außer in der Feinparfümerie beispielsweise auch zur Parfümierung von Seifen, Reinigungs- und Waschmitteln oder Weichspülern dienen kann.

In eigenen Untersuchungen zeigte 3-(4-Methyl-cyclohex-3-enyl)-butyraldehyd (Limonenal) einen sehr intensiven, modernen, frischen, grünen, aldehydigen, etwas fettigen und citrusartigen Geruch, der mit blumig-fruchtigen Noten gepaart ist. Der grüne citrusartige Geruch erinnert an die Schale von Citrusfrüchten, im speziellen Zitronen und Limonen. Die fettige Geruchsnote wird jedoch in zunehmenden Maße als störend empfunden, da sie die Einsetzbarkeit des Limonenal in Riechstoffmischungen (insbesondere Parfümkompositionen) beschränkt.

EP 0 857 723 beschreibt die Herstellung und Verwendung cyclischer Cedren-Acetale, u.a. Ambrocenide®. Allgemein besitzen die dort beschriebenen Verbindungen Geruchseigenschaften vom Ambratyp und vermitteln gleichzeitig einen strahlenden, starken Effekt, der ganz unterschiedliche Parfümnoten verstärkt und ihre Duftwirkung verlängert. Eine Mischung aus 3-(4-Methyl-cyclohex-3-enyl)-butyraldehyd (Limonenal) mit Ambrocenide® wird jedoch nicht offenbart.

Hinweise auf eine geruchsmarkierende Wirkung von Ambrocenide® wurden bislang nicht veröffentlicht.

Es war die primäre Aufgabe der vorliegenden Erfindung, eine Substanz (Mischung oder Einzelverbindung) anzugeben, welche die fettigen Geruchsnoten des Limonenal teilweise oder vollständig maskiert.

Es war insbesondere die Aufgabe der vorliegenden Erfindung, eine Substanz (Mischung oder Einzelverbindung) anzugeben, deren Geruchscharakteristik der von Limonenal sehr ähnlich ist, jedoch die fettigen Geruchsnoten des Limonenal nicht oder nur in abgeschwächter Form umfasst.

Vorzugsweise sollte die gestellte Aufgabe durch Angabe eines Maskierungsmittels für die fettige Geruchsnote des Limonenal gelöst werden.

Erfindungsgemäß wird die gestellte Aufgabe gemäß einem ersten Aspekt gelöst durch eine Mischung umfassend oder bestehend aus 3-(4-Methyl-cyclohex-3-enyl)-butyraldehyd (Limonenal)
und
4aR,5R,7aS,9R)-Octahydro-2,2,5,8,8,9a-hexamethyl-4H-4a,9-Methanoazuleno(5,6-d)-1,3-dioxol (Ambrocenide),
wobei das Gewichtsverhältnis von Limonenal zu Ambrocenide im Bereich von 150 : 1 bis 2500 : 1 liegt.

Überraschenderweise besitzen erfindungsgemäße Mischungen einen Geruch, der trotz des großen Gewichtsanteils von Limonenal keinen oder nur noch kaum spürbare fettige Geruchsnoten umfasst. Überraschenderweise kann Ambrocenide® die fettige Geruchsnote des Limonenal maskieren, ohne in den erfindungsgemäßen Mischungen mit Geruchsnoten vom Ambratyp einen störenden Einfluss zu nehmen. Entscheidend für den parfümistischen Erfolg (Maskierung ohne Einbringen dominanter Ambranoten) ist hierbei das eingestellte Gewichtsverhältnis von Limonenal zu Ambrocenide®, das im Bereich vom 600 : 1 bis 2500 : 1 liegen sollte. Besonders bevorzugt ist ein Gewichtsverhältnis von Limonenal zu Ambrocenide im Bereich von 800 : 1 bis 2500 : 1. Vergleiche hierzu auch die detaillierten Ausführungen weiter unten.

Die in erfindungsgemäßen Mischungen enthaltenen Verbindungen Limonenal und Ambrocenide® können dabei jeweils als reine Enantiomere oder als Mischungen von Enantiomeren vorliegen. Ambrocenide® kann dabei - wie oben bereits ausgeführt - in Form von einem, zwei, drei oder sämtlichen oben dargestellten Diastereomeren vorliegen.

Bei Herstellung der erfindungsgemäßen Mischungen wird Ambrocenide® vorzugsweise, insbesondere zwecks einfacherer Handhabung und Dosierung, in Mischung mit Verdünnungs- oder Lösungsmitteln eingesetzt. Bevorzugt sind 1 - 50 Gew.-%ige Lösungen, weiter bevorzugt 5 bis 25 Gew.-%ige Lösungen von Ambrocenide®, vorzugsweise in parfümistisch akzeptablen Lösungsmitteln. Bevorzugte parfümistisch akzeptable Lösungsmittel sind Ethanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat.

Eine erfindungsgemäße Mischung umfasst dementsprechend vorzugsweise ein oder mehrere Verdünnungs- oder Lösungsmittel ausgewählt aus der Gruppe bestehend aus Ethanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat. Hierbei wiederum bevorzugt sind Ethanol, Diethylphthalat, Propylenglycol, Dipropylenglycol, Triethylcitrat, Benzylbenzoat und Isopropylmyristat.

Für die industrielle parfümistische Praxis besonders wertvoll sind erfindungsgemäße Mischungen, in denen das Gewichtsverhältnis von Ambrocenide® zur Gesamtmenge an dem oder den besagten Verdünnungs- oder Lösungsmitteln im Bereich von 1 : 99 bis 1 : 1 liegt, vorzugsweise im Bereich von 5 : 95 bis 1 : 3. Die bevorzugten Gewichtsverhältnisse liegen insbesondere dann vor, wenn neben Limonenal und Ambrocenide® keine weiteren oder nur noch eine geringe Zahl und/oder Menge weiterer Riechstoffe vorliegt. Sobald Zahl und Menge weiterer Riechstoffe beträchtlich ist, kann es im Einzelfall vorteilhaft sein, die Gesamtmenge an der oder den Verdünnungs- oder Lösungsmitteln über die bevorzugt angegebenen Mengen (Verhältnisse) hinaus zu erhöhen.

In eigenen Untersuchungen hat sich gezeigt, dass Mischungen von Limonenal und Ambrocenide® in den oben angegebenen erfindungsgemäßen (bevorzugten) Mengenverhältnissen einen Geruch besitzen, der dem des reinen Limonenal weitgehend entspricht, wobei jedoch die unerwünschten fettigen Noten des Limonenal weitgehend oder sogar vollständig unterdrückt (maskiert) sind. Aufgrund der Unterdrückung der fettigen Geruchsnoten erscheint der Geruch der erfindungsgemäßen Mischung ausgeprägt natürlich und geruchlich besonders komplex.

Ambrocenide® wurde bei den eigenen Untersuchungen als 10%ige Lösung in DPG eingesetzt.

Einzelheiten ergeben sich aus der folgenden Tabelle:

| Gewichtsanteile | | Geruchsnoten (0 = nicht wahrnehmbar bis | | | | | |
|---|---|---|---|---|---|---|---|
| Limonenal | Ambrocenide® | 6 = sehr stark wahrnehmbar) | | | | | |
| | | Frisch | Citrus | Grün | Fettig | Holz | Ambra |
| 100 | - | 4 | 6 | 3 | 4 | 0 | 0 |
| 3500 | 1 | 4 | 6 | 3 | 4 | 0 | 1 |
| 1990 | 1 | 4 | 6 | 3 | 1 | 0 | 1 |
| 990 | 1 | 4 | 6 | 3 | 0 | 1 | 1 |
| 190 | 1 | 2 | 3 | 1 | 0 | 5 | 4 |
| 90 | 1 | 1 | 2 | 0 | 0 | 6 | 5 |
| - | 100 | 0 | 0 | 0 | 0 | 6 | 5 |

Man erkennt, dass insbesondere bei Gewichtsverhältnissen von Limonenal zu Ambrocenide® von 990 : 1 und 1990 : 1 eine vollständige bzw. zumindest nahezu vollständige Unterdrückung der fettigen Aldehydnote des Limonenal erreicht wird. Bei Gewichtsverhältnissen von 990 : 1 und 1990 : 1 wurden überdies nur sehr schwache Ambra-Noten wahrgenommen. Die sensorische Beurteilung der Mischungen mit Gewichtsverhältnissen von 990 : 1 und 1990 : 1 war insgesamt die vorteilhafteste.

Vorzugsweise umfasst eine erfindungsgemäße Mischung kein 3-(4-Methylcyclohexyl)-butyraldehyd, wie es üblicherweise bei der Herstellung von Limonenal aus Limonen als Nebenprodukt gebildet wird.

Eine erfindungsgemäße Mischung ist vorzugsweise eine Riechstoffmischung (z.B. Parfümkomposition), die neben Limonenal und Ambrocenide® mindestens einen gleichen Gewichtsanteil eines oder mehrerer anderer Riechstoffe umfasst. Insbesondere kann es sich bei einer solchen Riechstoffmischung um eine Parfümkomposition (ein Parfümöl) handeln.

Erfindungsgemäße Mischungen und insbesondere erfindungsgemäße Parfümkompositionen können in eine Reihe von Produkten eingearbeitet bzw. auf solche Produkte appliziert werden. Die Erfindung betrifft daher auch Produkte umfassend eine erfindungsgemäße Mischung.

Insbesondere sind erfindungsgemäße Mischungen für den Einsatz in tensidhaltigen Produkten geeignet. Gesucht werden nämlich - insbesondere für die Parfümierung von tensidhaltigen Formulierungen wie zum Beispiel Reinigungsmitteln (insbesondere Geschirrspülmittel und Allzweckreiniger) - häufig Riechstoffmischungen mit einer Citruskopfnote und ausgeprägter Natürlichkeit, wobei fettige Noten unerwünscht sind.

In erfindungsgemäßen Mischungen wird zudem eine fixierende Wirkung von Ambrocenide® auf Limonenal beobachtet. Im Vergleich zu reinem Limonenal kann mit einer erfindungsgemäßen Mischung nicht nur eine Citruskopfnote mit ausgeprägter Natürlichkeit ohne störende fettige Note erreicht werden, sondern gleichzeitig auch eine höhere Haftfestigkeit. Fixateure (wie vorliegend Ambrocenide) erhöhen die Haftfestigkeit von Riechstoffen (wie vorliegend Limonenal), sei es durch deren Dampfdruckerniedrigung oder mittels geruchlicher Verstärkung der (anderen) Riechstoffe (z.B. Absenkung des Schwellenwertes).

Die erfindungsgemäßen Mischungen sind zum Einsatz in tensidhaltigen Produkten besonders gut geeignet.

Bevorzugt ist es, wenn das (vorzugsweise tensidhaltige) Produkt eines der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -Iotionen, Handcremes und -Iotionen, Fußcremes und -lotionen, Enthaarungscremes und -lotionen, After -shave-Cremes und - lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder und Produkten der dekorativen Kosmetik.

Zutaten, mit denen die erfindungsgemäßen Mischungen kombiniert werden können, sind beispielsweise:
Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Die vorliegende Erfindung betrifft auch die Verwendung von Ambrocenide® zur Maskierung der fettigen Geruchsnote von Limonenal.

Ein entsprechendes erfindungsgemäßes Verfahren zur Maskierung der fettigen Geruchsnote von Limonenal umfasst den folgenden Schritt:

Mischen von Limonenal mit einer die fettige Geruchsnote des Limonenal teilweise oder vollständig maskierenden Menge von Ambrocenide®.

Es versteht sich, dass die eingesetzte Menge an Ambrocenide® in einem erfindungsgemäßen Verfahren vorzugsweise so ausgewählt ist, dass die holzige und/oder die Ambra-Note des Ambrocenide® gegenüber der frischen und/oder der Citrusnote des Limonenal nicht dominiert.

Besonders gute Maskierungserfolge werden erzielt, wenn das Gewichtsverhältnis von Limonenal zu Ambrocenide® in der resultierenden Mischung auf einen Wert im Bereich von 600 : 1 bis 2500 : 1, vorzugsweise im Bereich von 800 : 1 bis 2500 : 1 eingestellt wird. Bei Einstellung der bevorzugten Gewichtsverhältnisse wird sichergestellt, dass die holzige und/oder die Ambra-Note des Ambrocenide® nicht gegenüber den frischen und/oder Citrusnoten des Limonenal dominiert.

Mit den erfindungsgemäßen Verfahren werden vorzugsweise bevorzugte erfindungsgemäße Mischungen hergestellt.

Eine erfindungsgemäße Mischung bzw. eine nach einem erfindungsgemäßen Verfahren hergestellte Mischung lässt sich hervorragend als Riechstoff mit frischer, citrusartiger Note einsetzen, denn im Unterschied zu dem Einsatz von reinem Limonenal tritt dessen fettige Geruchsnote nicht in störendem Umfang auf.

Eine erfindungsgemäße Mischung kann, insbesondere bei einem Gewichtsverhältnis von Limonenal zu Ambrocenide® im Bereich von 600 : 1 bis 2500 : 1, als Riechstoffmischung zur Erzeugung eines intensiven natürlichen citrischen Geruchs dienen.

Ein entsprechendes erfindungsgemäßes Verfahren zum Versehen von (a) Haaren oder (b) textilen Fasern mit dem komplexen Geruchseindruck eines natürlichen citrischen Geruchs umfasst die folgenden Schritte:
- Bereitstellen einer erfindungsgemäßen Mischung
- Applizieren der Mischung auf das Haar oder die textilen Fasern.

Eine für die erfindungsgemäßen Verwendungen bzw. die entsprechenden Verfahren besonders geeignete Mischung ist eine Lösung, die umfasst:
(a) Wasser,
(b) Ambrocenide® und Limonenal in den oben genannten (bevorzugten) Gewichtsverhältnissen,
   sowie
(c) ein oder mehrere Tenside,
wobei die Konzentration an Limonenal in der Lösung im Bereich von 10⁻⁷ bis 10⁻¹ Gew.-% liegt. Weitere Riechstoffe und/oder sonstige übliche Zusatzstoffe können vorhanden sein.

Beispiele für Riechstoffe, mit denen eine erfindungsgemäße Mischung vorteilhaft kombiniert werden kann, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 4rd. Ed., Wiley-VCH, Weinheim 2001.

Bevorzugte erfindungsgemäße Parfümkompositionen (Parfümöle) umfassen neben Limonenal und Ambrocenide® (in den angegebenen Mengenverhältnissen) zumindest einen gleichen Gewichtsanteil eines oder mehrerer anderer Riechstoffe. Bevorzugte erfindungsgemäße Mischungen (insbesondere Parfümkompositionen) umfassen somit vorzugsweise neben Limonenal und Ambrocenide® einen gleichen oder höheren Gewichtsanteil an einem oder mehreren anderen Riechstoffen.

Erfindungsgemäße Parfümkompositionen umfassen neben Limonenal und Ambrocenide® vorzugsweise ein, zwei, drei, vier, fünf, sechs, sieben, acht, neun, zehn oder mehr weitere Riechstoffe.

Erfindungsgemäße Parfümkompositionen, die Limonenal und Ambrocenide® in den erfindungsgemäßen Gewichtsverhältnis umfassen, können in konzentrierter Form, in Lösungen oder in den unten beschriebenen modifizierten Formen verwendet werden z.B. für die Parfümierung von sauren, alkalischen und neutralen Reinigungsmitteln, wie z.B. pulver- und schaumförmigen Teppichreinigern, flüssigen Waschmitteln, pulverförmigen Waschmitteln, Wäschevorbehandlungsmitteln wie Bleichmittel, Einweichmittel und Fleckenentferner, Wäscheweichspülern, Waschseifen, Waschtabletten, sowie Körperpflegemitteln wie z.B. festen und flüssigen Seifen, Duschgelen, Shampoos, kosmetischen Emulsionen vom Öl-in-Wasser-, vom Wasser-in-Öl- und vom Wasser-in-Öl-in-Wasser-Typ Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen.

Erfindungsgemäße Parfümkompositionen, die Limonenal und Ambrocenide® in den erfindungsgemäßen Gewichtsverhältnissen umfassen, können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Erfindungsgemäße Mischungen und insbesondere erfindungsgemäße Parfümkompositionen können an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Erfindungsgemäße Mischungen und insbesondere erfindungsgemäße Parfümkompositionen können auch mikroverkapselt, sprühgetrocknet, als Einschluss-Komplexe oder als Extrusions-Produkte vorliegen und in dieser Form dem zu parfümierenden Produkt hinzugefügt werden.

Gegebenenfalls können die Eigenschaften derart modifizierter erfindungsgemäßer Mischungen, insbesondere derart modifizierter erfindungsgemäßer Parfümkompositionen (Parfümöle), durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung von erfindungsgemäßen Mischungen, insbesondere erfindungsgemäßer Parfümkompositionen, kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrine und pflanzliche Gummen verwendet werden können. Einschluss-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Bevorzugte Produkte, die im Rahmen der vorliegenden Erfindung eingesetzt werden können, sind (a) Parfümöle für tensidhaltige Formulierungen, wie z.B. Reinigungsmittel, Waschmittel, Weichspüler sowie Körperpflegemittel sowie (b) die entsprechenden tensidhaltigen Formulierungen selbst.

Die tensidhaltigen Formulierungen, die im Rahmen der vorliegenden Erfindung eingesetzt werden können, umfassen allgemein Substanzen aus der Klasse der anionischen Tenside, wie zum Beispiel Carboxylate, Sulfate, Sulfonate und Phosphate, der kationischen Tenside, wie zum Beispiel quartäre Ammoniumsalze, der amphoteren Tenside, wie zum Beispiel Betaine, und der nichtionischen Tenside, wie zum Beispiel Ethoxylate und Propoxylate.

Unter den anionischen Tensiden sind die Sulfate und Sulfonate bevorzugt. Bei den Sulfaten sind solche mit 12 bis 18 Kohlenstoffatomen und einem Ethoxylierungsgrad von 1 bis maximal 5 bevorzugt. Insbesondere bevorzugt ist Natriumlaurylethersulfat, vorzugsweise mit einem mittleren Ethoxylierungsgrad von 2 bis 4.

Unter den Sulfonaten sind insbesondere die linearen Natriumalkylbenzolsulfonate mit durchschnittlich ca. 12 Kohlenstoffatomen in der Alkylkette, wobei diese aus homologen Resten mit 10 bis 14 Kohlenstoffatomen bestehen ("Dodecylbenzolsulfonat"), bevorzugt.

Aus der Gruppe der nichtionischen Tenside sind die ethoxylierten Fettalkohole, die durch Ethoxylierung von Alkoholen mit 12 bis 18 Kohlenstoffatomen erhalten werden (Fettalkoholethoxylate mit 12 bis 18 C-Atomen), bevorzugt. Der Ethoxylierungsgrad kann dabei in weiten Grenzen variieren, besonders bevorzugt sind jedoch Produkte mit einem durchschnittlichen Ethoxylierungsgrad von 5 bis 10, insbesondere von 7 Mol addiertem Ethylenoxid pro Mol Fettalkohol.

Unter den Betainen sind insbesondere solche vom Säureamidtyp mit der aufgeführten Struktur bevorzugt.

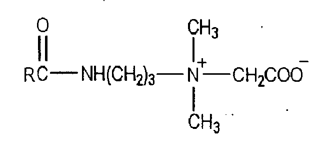

Ein bevorzugter Rest RC=0 ist dabei der Schnitt aus den Fettsäuren des Kokosöls, in dem die Laurylsäure mit 45-50% der Hauptbestandteil ist.

In Kombination mit ausgewählten Tensiden sind die günstigen Eigenschaften der erfindungsgemäßen Mischungen, insbesondere der erfindungsgemäßen Parfümkompositionen (Parfümöle) besonders stark ausgeprägt. Ein entsprechendes erfindungsgemäßes tensidhaltiges Produkt umfasst vorzugsweise neben Limonenal und Ambrocenide® (in den erfindungsgemäßen Gewichtsverhältnissen) ein oder mehrere Tenside, die ausgewählt sind aus der Gruppe bestehend aus:
- Lineare Alkylbenzolsulfonate (insbesondere die oben genannten, wie z.B. die linearen Natriumalkylbenzolsulfonate),
- Fettalkoholethoxylate mit 12-18 C-Atomen (insbesondere die oben genannten, also z.B. die mit dem oben als bevorzugt gekennzeichneten Ethoxylierungsgrad),
- Laurylethersulfate (insbesondere die oben genannten, also z.B. das oben genannte Natriumlaurylethersulfat) und
- Betaine (insbesondere die oben genannten, also z.B. Betaine vom Säureamidtyp mit der oben aufgeführten Struktur).

Lineare Alkylbenzolsulfonate und Fettalkoholethoxylate mit 12-18 C-Atomen werden dabei vorzugsweise nebeneinander eingesetzt, insbesondere in Vollwaschmittelpulvern.

Ebenso werden Laurylethersulfate (insbesondere das oben genannte Natriumlaurylethersulfat) und Betaine (insbesondere Betaine vom Säureamidtyp mit der oben aufgeführten Struktur) vorzugsweise nebeneinander eingesetzt, insbesondere in Feinwaschmitteln, Shampoos und Duschgelen.

Die Konzentration der oberflächenaktiven Stoffe in den erfindungsgemäßen tensidhaltigen Produkten ist in der Regel unkritisch. Bevorzugte Konzentrationen sind abhängig vom Typ des Tensids und der jeweiligen Anwendung. Sie können zum Beispiel in speziellen Bleichprodukten kleiner 1 Gew.-%, in Seifen oder Waschpulver aber größer 99 Gew.-% sein.

Für bestimmte Anwendungsgebiete sind in erfindungsgemäßen tensidhaltigen Produkten bestimmte Kombinationen und Konzentrationen bevorzugt. So sind erfindungsgemäße Mischungen (Waschmittelformulierungen) bevorzugt, in denen der Anteil an linearen Alkylbenzolsulfonaten im Bereich von 7 - 10 Gew.-% und/oder der Anteil an Fettalkoholethoxylaten mit 12-18 C-Atomen im Bereich von 3 - 6 Gew.-% liegt, jeweils bezogen auf die Gesamtmasse der Mischung. Zudem sind erfindungsgemäße Mischungen (Formulierungen für Feinwaschmittel, Shampoos und Duschgele) bevorzugt, in denen der Anteil an Natriumlaurylethersulfat im Bereich von 7 - 13 Gew.-% und/oder der Anteil an Betain (insbesondere Betain vom Säureamidtyp mit der oben aufgeführten Struktur) im Bereich von 1 - 3 Gew.-% liegt, jeweils bezogen auf die Gesamtmasse der Mischung.

Insbesondere sofern die tensidhaltigen Produkte zur Behandlung von Haaren oder textilen Fasern vorgesehen sind, ist eine weitere wichtige anwendungstechnische Anforderung die Substantivität gegenüber dem bzw. Retention am Substrat, insbesondere also Haaren oder textilen Fasern. Die Bedeutung der Substantivität und der Retention wird z.B. in EP 1 201 738 A1 ausführlich dargelegt, vgl. dort die Abschnitte [0004]-[0005].

Die eine erfindungsgemäße Mischung enthaltenden tensidhaltigen Produkte zeigen ferner eine überraschend erhöhte Substantivität bzw. Retention gegenüber Haar, Wolle, Baumwolle sowie anderen Textilfasern.

Die folgenden Beispiele dienen der Erläuterung der Erfindung. Sofern nicht anders angegeben beziehen sich alle Angaben auf das Gewicht.

Das in den folgenden Beispielen eingesetzte Ambrocenide® wurde ausgehend von (-)-alpha-Cedrenoxid analog den Beispielen 1, 4 und 5 der EP 0 857 723 hergestellt. Androcenide® ist ein Handelsprodukt der Symrise GmbH & Co. KG.

Beispiel 1: Parfümöl, insbesondere geeignet für die Einarbeitung in ein Shampoo.

| | Gew.-teile | Gew.-teile |
|---|---|---|
| | **A** | **B** |
| Allylamylglycolat | 4,00 | 4,00 |
| Bergamottöl, furocumarinfrei | 80,00 | 80,00 |
| Calone 1951 (7-Methyl-2H-1,5-benzodioxepin-3(4H)-on) | 1,00 | 1,00 |
| Canthoxal (2-Methyl-3-(4-methoxyphenyl)propanal) | 2,00 | 2,00 |
| Cassis 345B | 2,00 | 2,00 |
| Cedernblätteröl | 2,00 | 2,00 |
| Cedernholzöl | 5,00 | 5,00 |
| Cypressenöl | 7,00 | 7,00 |
| Damascon alpha | 0,50 | 0,50 |
| Dihydromyrcenol | 150,0 | 150,0 |
| Eugenol | 8,00 | 8,00 |
| Evernyl (2,4-Dihydroxy-3,6-dimethylbenzoesäuremethyl-ester) | 0,50 | 0,50 |
| Fleursandol ((E)-4-(3a,4,5,6,7,7a-Hexahydro-3H-4,7-methano-inden-5-yl)-3-methyl-but-3-en-2-ol) | 40,00 | 40,00 |
| Florazon (3-(4-Ethylphenyl)-2,2-dimethylpropanal) | 5,00 | 5,00 |
| Galaxolid® 50% in DEP (4,6,6,7,8,8-Hexamethyl-1,3,4,6,7,8-hexahydrocyclopenta[g]benzopyran) | 35,00 | 35,00 |
| Grapefruit Base | 3,00 | 3,00 |
| Hedion® (Methyldihydrojasmonat) | 288,0 | 290,0 |
| Helional® (2-Methyl-3-(3,4-metylendioxophenyl)propanal) | 60,00 | 60,00 |
| Hexenol cis-3 | 2,00 | 2,00 |
| Iso E Super® (2,3,8,8-Tetramethyl-1,2,3,4,5,6,7,8-octahydro-2-naphthalenylmethylketon) | 50,00 | 50,00 |
| Lavandinöl Abrialis, natürlich | 15,00 | 15,00 |
| Lemon Oil | 20,00 | 20,00 |
| Vertocitral (2,4-Dimethyl-3-cyclohexencarbaldehyd) | 2,00 | 2,00 |
| Lilial® (2-Methyl-3-(4-tert-butylphenyl)propanal) | 20,00 | 20,00 |
| Linalool | 80,00 | 80,00 |
| Mandarinenöl | 20,00 | 20,00 |
| Orangenöl brasilianisch | 15,00 | 15,00 |
| Patchuliöl | 10,00 | 10,00 |
| Rosmarinöl, spanisch | 5,00 | 5,00 |
| Salbeiöl dalmatisch | 3,00 | 3,00 |
| Sandranol® | 5,00 | 5,00 |
| Tonalid® (1-(5,6,7,8-Tetrahydro-3,5,5,6,8,8-hexamethyl-2-naphthalenyl)ethanon) | 10,00 | 10,00 |
| 3-(4-Methyl-cyclohex-3-enyl)-butyraldehyd (Limonenal) | 47,50 | 47,50 |
| Ambrocenide® 10%ig in DPG | 2,50 | 0,50 |
| TOTAL | 1000,00 | 1000,00 |

| | | |
|---|---|---|
| Abkürzungen: DEP = Diethylphthalat, DPG: Dipropylenglycol | | |

In Vergleich mit einer Mischung, die anstelle der insgesamt 47,50 Gewichtsteile Limonenal und der 0,50 Gewichtsteile Ambrocenide® 10%ig in DPG 48 Gewichtsteile DPG enthielt, wies das obige Limonenal und Ambrocenide® enthaltende Parfümöl eine merklich intensiver citrische, weichere und komplexere Note auf, wobei die gesamte Komposition harmonischer wirkte. Fettige Noten wurden nicht festgestellt.

In Vergleich mit einer Mischung, die anstelle der insgesamt 47,50 Gewichtsteile Limonenal und der 2,50 Gewichtsteile Ambrocenide® 10%ig in DPG 50 Gewichtsteile DPG enthielt, wies das obige Limonenal und Ambrocenide® enthaltende Parfümöl eine citrischere, wärmere, komplexere und leicht holzig-ambrierte Note auf, wobei die gesamte Komposition harmonischer wirkte. Fettige Noten wurden nicht festgestellt.

### Beispiel 2: Shampoo

Die Parfümölkompositionen A und B aus Beispiel 1 wurden separat in einer Dosierung von 0,4 Gew.-% in eine Shampoo-Grundmasse folgender Zusammensetzung eingearbeitet:

| | |
|---|---|
| Natriumlaurylethersulfat | 12% |
| (z.B. Texapon NSO, Fa. Cognis Deutschland GmbH) | |
| Cocamidopropylbetain | 2% |
| (z.B. Dehyton K, Fa. Cognis Deutschland GmbH) | |
| Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus werden 100 mL einer 20 Gew. %-igen wässrigen Shampoo-Lösung (als Beispiel einer erfindungsgemäßen Lösung) hergestellt. In dieser Shampoo-Lösung werden 2 Haarsträhnchen gemeinsam für 2 Minuten gewaschen und anschließend 20 Sekunden unter fließendem handwarmen Wasser gespült. Eine Haarsträhne wird nass in Aluminiumfolie eingepackt und die zweite Haarsträhne mit einem Fön getrocknet. Beide Haarsträhnen werden von einem Panel geruchlich beurteilt.

### Beispiel 3: Weichspüler

Die Parfümölkompositionen A und B aus Beispiel 1 wurden separat in einer Dosierung von 0,4 Gew.-% in eine Weichspüler-Grundmasse folgender Zusammensetzung eingearbeitet:

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer AG) | |
| Farblösung, ca. 1%-ig | 0,3% |
| Wasser | 94,0% |

Der pH-Wert der Weichspüler-Grundmasse lag im Bereich 2 - 3. Zwei Stofflappen werden mit 370 g einer aus der Grundmasse hergestellten 1 %-igen wässrigen Weichspüler-Lösung (als Beispiel einer erfindungsgemäßen Lösung) in einer Linetest-Maschine im Weichspülprogramm 30 Minuten bei 20°C gespült. Die Lappen werden ausgewrungen und anschließend 20 Sekunden geschleudert. Ein Lappen wird nass eingeschweißt, und einer zum Trocknen aufgehängt. Anschließend werden beide Lappen durch ein Panel geruchlich beurteilt.

### Beispiel 4: Waschpulver

Die Parfümölkompositionen A und B aus Beispiel 1 wurden separat in einer Dosierung von 0,3 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis (methylen)]]tetrakis-phosphonsäure, Natriumsalz) Optischer Aufheller | 0,2 % |
| Na-Sulfat | 6,5 % |
| Protease | 0,4 % |
| Natriumperborattetrahydrat | 22,0 % |
| TAED | 1,0 % |

Zwei Stofflappen werden mit 370 g einer aus der Grundmasse hergestellten 1%-igen wässrigen Waschpulverlauge (als Beispiel einer erfindungsgemäßen tensidhaltigen Lösung; der pH-Wert der Waschpulverlauge liegt deutlich im basischen Bereich) in einer Linetest-Maschine im Hauptwaschgang 45 Minuten bei 60°C gewaschen. Die Lappen werden zunächst 5 Minuten mit kaltem Wasser gespült, ausgewrungen und anschließend 20 Sekunden geschleudert. Ein Lappen wird nass eingeschweißt, und einer zum Trocknen aufgehängt. Anschließend werden beide Lappen durch ein Panel geruchlich beurteilt.

## Patentansprüche

1. Mischung umfassend oder bestehend aus
3-(4-Methyl-cyclohex-3-enyl)-butyraldehyd (Limonenal)
und
4aR,5R,7aS,9R)-Octahydro-2,2,5,8,8,9a-hexamethyl-4H-4a,9-Methanoazuleno(5,6-d)-1,3-dioxol (Ambrocenide),
wobei das Gewichtsverhältnis von Limonenal zu Ambrocenide im Bereich von 150 : 1 bis 2500 : 1 liegt.

2. Mischung nach Anspruch 1,
wobei das Gewichtsverhältnis von Limonenal zu Ambrocenide im Bereich von 600 : 1 bis 2500 : 1, vorzugsweise im Bereich von 800 : 1 bis 2500 : 1, liegt.

3. Mischung nach einem der vorangehenden Ansprüche, weiter umfassend ein oder mehrere Verdünnungs- oder Lösungsmittel ausgewählt aus der Gruppe bestehend aus Ethanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat.

4. Mischung nach Anspruch 3, wobei das Gewichtsverhältnis von Ambrocenide zur Gesamtmenge an dem oder den besagten Verdünnungs- oder Lösungsmitteln im Bereich von 1 : 99 bis 1 : 1 liegt, vorzugsweise im Bereich von 5 : 95 bis 1 : 3.

5. Mischung nach einem der vorangehenden Ansprüche, wobei die Mischung eine Riechstoffmischung ist, die neben Limonenal und Ambrocenide mindestens einen gleichen Gewichtsanteil eines oder mehrerer anderer Riechstoffe umfasst.

6. Verwendung von Ambrocenide zur Maskierung der fettigen Geruchsnote von Limonenal.

7. Verfahren zur Maskierung der fettigen Geruchsnote von Limonenal, mit folgendem Schritt:
- Mischen von Limonenal mit einer die fettige Geruchsnote des Limonenal teilweise oder vollständig maskierenden Menge von Ambrocenide.

8. Verfahren nach Anspruch 7, wobei die eingesetzte Menge an Ambrocenide so ausgewählt ist, dass die holzige und/oder die Ambra-Note des Ambrocenide gegenüber der frischen und/oder der Citrusnote des Limonenal nicht dominiert.

9. Verfahren nach einem der Ansprüche 7 bis 8, wobei das Gewichtsverhältnis von Limonenal zu Ambrocenide in der Mischung auf einen Wert im Bereich von 150 : 1 bis 2500 : 1, vorzugsweise im Bereich von 600 : 1 bis 2500 : 1 eingestellt wird.

10. Verwendung einer Mischung nach einem der Ansprüche 2 bis 5 als Riechstoff mit frischer, citrusartiger Note.

## Claims

1. A mixture comprising or consisting of
3-(4-methyl-cyclohex-3-enyl)-butyraldehyde (limonenal)
and
4aR,5R,7aS,9R)-octahydro-2,2,5,8,8,9a-hexamethyl-4H-4a,9-methanoazuleno(5,6-d)-1,3-dioxol (ambrocenide),
wherein the weight ratio of limonenal to ambrocenide is in the range of from 150:1 to 2500:1.

2. The mixture as claimed in Claim 1,
wherein the weight ratio of limonenal to ambrocenide is in the range of from 600:1 to 2500:1, preferably in the range of from 800:1 to 2500:1.

3. The mixture as claimed in any one of the preceding claims, further comprising one or more diluents or solvents selected from the group consisting of ethanol, dipropylene glycol (DPG), propylene glycol, 1,2-butylene glycol, glycerin, diethylene glycol monoethyl ether, diethyl phthalate (DEP), isopropyl myristate (IPM), triethyl citrate (TEC), benzyl benzoate (BB) and benzyl acetate.

4. The mixture as claimed in Claim 3, wherein the weight ratio of ambrocenide to the total quantity of said diluents(s) or solvent(s) is in the range of from 1:99 to 1:1, preferably in the range of from 5:95 to 1:3.

5. The mixture as claimed in any one of the preceding claims, wherein the mixture is an aromatic substance mixture, which besides limonenal and ambrocenide comprises at least an equal proportion by weight of one or more other aromatic substances.

6. A use of ambrocenide for masking the fatty olfactory note of limonenal.

7. A process for masking the fatty olfactory note of limonenal, having the following step:
- mixing limonenal with a quantity of ambrocenide that partly or fully masks the fatty olfactory note of the limonenal.

8. The process as claimed in Claim 7, wherein the quantity of ambrocenide used is selected such that the woody and/or ambergris note of the ambrocenide does not dominate over the fresh and/or citrus note of the limonenal.

9. The process as claimed in any one of claims 7 to 8, wherein the weight ratio of limonenal to ambrocenide in the mixture is set to a value in the range of from 150:1 to 2500:1, preferably in the range of from 600:1 to 2500:1.

10. A use of a mixture as claimed in any one of the claims 2 to 5 as an aromatic substance with a fresh, citrus-like note.

## Revendications

1. Mélange comprenant ou formé de
3-(4-méthyl-cyclohex-3-ényl)-butyraldéhyde (limonène)
et
(4aR,5R,7aS,9R)-octahydro-2,2,5,8,8,9a-hexaméthyl-4H-4a,9-méthanoazulène(5,6-d)-1,3-dioxol (ambrocénide),
le rapport pondéral entre le limonène et l'ambrocénide étant compris entre 150:1 et 2500:1.

2. Mélange selon la revendication 1, le rapport pondéral entre le limonène et l'ambrocénide étant compris entre 600:1 et 2500:1, de préférence entre 800:1 et 2500:1.

3. Mélange selon l'une des revendications précédentes, comprenant, en outre, un ou plusieurs agent/s de dilution ou solvant/s choisi/s dans le groupe comprenant l'éthanol, le dipropylène glycol (DPG), le propylène glycol, le 1,2-butylène glycol, la glycérine, le diéthylène glycol monoéthyl éther, le diéthylphtalate (DEP), l'isopropylmyristat (IPM), le triéthylcitrate (TEC), le benzylbenzoate (BB) et le benzylacétate.

4. Mélange selon la revendication 3, le rapport pondéral entre l'ambrocénide et la quantité totale du ou des agent/s de dilution ou solvant/s mentionné/s étant compris entre 1:9 et 1:1, de préférence entre 5:95 et 1:3.

5. Mélange selon l'une des revendications précédentes, le mélange étant un mélange de matières odorantes qui comprend, outre le limonène et l'ambrocénide, au moins une teneur pondérale identique en une ou plusieurs autre/s matière/s odorante/s.

6. Utilisation de l'ambrocénide pour masquer la note olfactive grasse du limonène.

7. Procédé pour masquer la note olfactive grasse du limonène, comprenant l'étape suivante :
- mélange du limonène avec une quantité d'ambrocénide masquant partiellement ou totalement la note olfactive grasse du limonène.

8. Procédé selon la revendication 7, la quantité d'ambrocénide utilisée étant choisie de manière à ce que la note boisée et/ou ambrée de l'ambrocénide ne domine pas par rapport la note fraîche et/ou la note citronnée du limonène.

9. Procédé selon l'une des revendications 7 à 8, le rapport pondéral entre le limonène et l'ambrocénide dans le mélange étant fixé à une valeur comprise entre 150:1 et 2500:1, de préférence entre 600:1 et 2500:1.

10. Utilisation d'un mélange selon l'une des revendications 2 à 5 en tant que matière odorante à note fraîche, citronnée.
